# EUROPEAN PATENT SPECIFICATION

(11) **EP 4 136 121 B1**
(45) Date of publication and mention of the grant of the patent: **28.05.2025**
(21) Application number: 21720575.6
(22) Date of filing: 15.04.2021
(51) Int. Cl.: C07K 16/46, B01D 15/36, G01N 30/34, G01N 30/88

(54) **SYSTEMS, MATERIALS, AND METHODS FOR REVERSED-PHASE HIGH PERFORMANCE LIQUID CHROMATOGRAPHY (RP-HPLC) FOR MONITORING FORMATION OF MULTI-SPECIFIC MOLECULES**
SYSTEME, MATERIALIEN UND METHODEN FÜR DIE UMKEHRPHASEN-HOCHLEISTUNGSFLÜSSIGKEITSCHROMATOGRAPHIE ZUR ÜBERWACHUNG DER BILDUNG MULTISPEZIFISCHER MOLEKÜLE
SYSTÈMES, MATÉRIAUX ET PROCÉDÉS DE CHROMATOGRAPHIE LIQUIDE À HAUTE PERFORMANCEEN PHASE INVERSE POUR SURVEILLER LA FORMATION DE MOLÉCULES MULTISPÉCIFIQUES

(30) Priority: 16.04.2020 US 202063010907 P; 16.04.2020 US 202063010912 P; 16.04.2020 US 202063010920 P; 16.04.2020 US 202063010926 P
(43) Date of publication of application: 22.02.2023
(73) Proprietor: Janssen Biotech, Inc., Horsham, PA 19044 (US)
(72) Inventor: MARTOSELLA, James, Malvern, PA 19355 (US); FELICIANO-CARDONA, Jessika S., Malvern, PA 19355 (US); ELOVE, Gulnur, Malvern, PA 19355 (US)
(74) Representative: Carpmaels & Ransford LLP
(86) International application number: PCT/IB2021/053127
(87) International publication number: WO 2021/209953

(56) References cited:
- EP-A2- 1 645 564
- US-A1- 2005 161 399
- ANONYMOUS: "POROS(TM) R2 20 [mu]m Column, Stainless Steel, 4.6 x 100 mm, 1.7 mL", 16 May 2024 (2024-05-16), pages 1 - 5, XP093163862, Retrieved from the Internet <URL:https://www.thermofisher.com/order/catalog/product/es/en/1112426>
- DIMASI NAZZARENO ET AL: "Guiding bispecific monovalent antibody formation through proteolysis of IgG1 single-chain", MABS, vol. 9, no. 3, 3 April 2017 (2017-04-03), US, pages 438 - 454, XP055822615, ISSN: 1942-0862, Retrieved from the Internet <URL:https://www.ncbi.nlm.nih.gov/pmc/articles/PMC5384712/pdf/kmab-09-03-1277301.pdf> DOI: 10.1080/19420862.2016.1277301
- GIESE GLEN ET AL: "Bispecific antibody process development: Assembly and purification of knob and hole bispecific antibodies", BIOTECHNOLOGY PROGRESS, vol. 34, no. 2, 17 January 2018 (2018-01-17), pages 397 - 404, XP055822613, ISSN: 8756-7938, Retrieved from the Internet <URL:https://api.wiley.com/onlinelibrary/tdm/v1/articles/10.1002%2Fbtpr.2590> DOI: 10.1002/btpr.2590
- MANIKWAR PRAKASH ET AL: "Characterization of a Novel Bispecific Antibody With Improved Conformational and Chemical Stability", JOURNAL OF PHARMACEUTICAL SCIENCES, vol. 109, no. 1, 7 July 2019 (2019-07-07), US, pages 220 - 232, XP055794177, ISSN: 0022-3549, Retrieved from the Internet <URL:https://www.jpharmsci.org/article/S0022-3549(19)30428-9/pdf> DOI: 10.1016/j.xphs.2019.06.025
- JOHN DOLAN: "A Guide to HPLC and LC-MS Buffer Selection", HPLC COLUMNS, 5 July 2006 (2006-07-05), XP055318419, Retrieved from the Internet <URL:https://www.researchgate.net/file.PostFileLoader.html?id=57602f96b0366de7491f6a17&assetKey=AS:372915662016512@1465921430626> [retrieved on 20161110]
- AMARAL MARTA ET AL: "Engineered Technologies and Bioanalysis of multispecific Antibody Formats", JOURNAL OF APPLIED BIOANALYSIS, vol. 6, no. 1, 31 January 2020 (2020-01-31), pages 26 - 51, XP055822537, Retrieved from the Internet <URL:https://betasciencepress-publishing.com/wp-content/uploads/1017145_jab_pdf/JAB20005-Engineered-Technologies-and-Bioanalysis-of-multispecific-Antibody-Formats.pdf> DOI: 10.17145/jab.20.005

## Description

### FIELD OF THE INVENTION

This invention relates to a high-pressure liquid chromatography (HPLC) method for detecting multi-specific molecule formation after a multi-specific molecule manufacturing or development process. The HPLC method provides fast, quantitative, real-time processing of multi-specific molecule formation.

### BACKGROUND OF THE INVENTION

During the manufacturing or process research and development (R&D) of a multispecific molecule (e.g., a multi-specific antibody), the potential for incomplete formation of the multi-specific molecule (e.g., a multi-specific antibody) remains regardless of the manufacturing process. Non-reduced capillary sodium dodecyl sulfate (NR cSDS) has previously been employed to monitor incomplete multi-specific antibody formation; however, the technique is not practical for delivering fast, quantitative, real-time results that are needed when studying multi-specific formation in a process manufacturing environment or during multi-specific molecule (e.g., multi-specific antibody) development. Thus, a fast, quantitative, real-time process monitoring multi-specific molecule (e.g., multi-specific antibody) formation is needed.

Dimasi N. et al., MABS, vol. 9, no. 3, 2017, pages 438-454 describes "an IgG1 domain-tethering approach to guide the correct assembly of 2 light and 2 heavy chains, derived from 2 different antibodies, to form bispecific monovalent antibodies in IgG1 format".

Giese G. et al., Biotechnology Progress, vol. 34, no. 2, 2018, pages 397-404 describes a "process for the assembly and purification of knob and hole dual light chain bispecific antibodies".

Manikwar P. et al., Journal of Pharmaceutical Sciences, vol. 109, no. 1, 2019, pages 220-232, describes "the stability of a novel bispecific format, BiS5, where the scFv is tethered to the C_{H}3 domain".

US 2005/0161399 A1 describes "a method of analysis of high molecular weight proteins".

Dolan J., A Guide to HPLC and LC-MS Buffer Selection, may be a "guide for buffer usage".

Amaral M. et al. Journal of Applied Bioanalysis, vol. 6, no. 1, 2020, pages 26-51 describes "the analytical methods available to characterize ... complex molecules, focusing on mispairing analysis and functional characterization".

EP1645564 A2 describes "a system and method for protein separation including: fractionating a mixture of proteins on a reversed-phase superficially porous stationary phase at a temperature of greater than or equal to about 40 °C to recover a protein in from about 70 to 100 weight percent of the mixture of proteins".

### BRIEF SUMMARY OF THE INVENTION

Embodiments of the present invention are set out in the enclosed claims.

In one embodiment, the present invention provides a method of detecting formation of a multi-specific molecule, the method comprising:
a. obtaining a multi-specific molecule sample;
b. obtaining a reversed-phase high performance liquid chromatography (RP-HPLC) column;
c. contacting the multi-specific molecule sample in a polar aqueous mobile phase A with the RP-HPLC column, wherein the polar aqueous mobile phase A is a solution comprising about 1% to about 5% isopropanol and an ion-pairing agent;
d. contacting an organic non-polar mobile phase B with the RP-HPLC column, wherein the organic non-polar mobile phase B is a solution comprising about 60% to about 80% isopropanol and about 15% to about 25% acetonitrile and an ion-pairing agent;
e. eluting the multi-specific molecule sample; and
f. monitoring the amount of multi-specific molecule formation in the eluted multispecific molecule sample.

In one embodiment, the present invention provides a system comprising a reversed-phase high performance liquid chromatography (RP-HPLC) member for detecting formation of a multi-specific molecule, the system comprising:
a. a multi-specific molecule sample in a polar aqueous mobile phase A, wherein the polar aqueous mobile phase A is a solution comprising about 1% to about 5% isopropanol and an ion pairing agent;
b. a RP-HPLC column;
c. an organic non-polar mobile phase B, wherein the organic non-polar mobile phase B is a solution comprising about 60% to about 80% isopropanol and about 15% to about 25% acetonitrile and an ion pairing agent;

wherein the RP-HPLC column is configured to be contacted with the multi-specific molecule sample in polar aqueous mobile phase A, and
wherein the RP-HPLC is further configured to be contacted with the organic non-polar mobile phase B to elute a multi-specific molecule sample, wherein an amount of multispecific molecule formation can be identified in the eluted multi-specific molecule sample.
In certain embodiments, the multi-specific molecule is a multi-specific antibody.

Also provided herein are methods of detecting formation of a multi-specific antibody while performing a manufacturing process to produce a multi-specific antibody. The methods comprise (a) performing a manufacturing process to produce a multi-specific antibody, wherein the manufacturing process results in a multi-specific antibody sample comprising an amount of a multi-specific antibody; (b) setting up a reversed-phase high performance liquid chromatography (RP-HPLC) column; (c) injecting the multi-specific antibody sample in a polar aqueous mobile phase A in the RP-HPLC column, wherein the polar aqueous mobile phase A is a solution comprising about 1% to about 5% isopropanol and an ion-pairing agent; (d) applying an organic non-polar mobile phase B to the RP-HPLC column to form a gradient elution, wherein the organic non-polar mobile phase B is a solution comprising about 60% to about 80% isopropanol and about 15% to about 25% acetonitrile and an ion-pairing agent; (e) eluting the multi-specific antibody sample; and (f) monitoring the amount of multi-specific antibody formation in the eluted sample.

Also provided herein are methods of detecting formation of a bispecific antibody while performing a controlled FAB arm exchange (cFAE) to produce a bispecific antibody. The methods comprise (a) performing a controlled FAB arm exchange (cFAE) to produce a cFAE sample, wherein the cFAE sample comprises an amount of the bispecific antibody; (b) setting up a reversed-phase high performance liquid chromatography (RP-HPLC) column; (c) injecting the cFAE sample in a polar aqueous mobile phase A in the RP-HPLC column, wherein the polar aqueous mobile phase A is a solution comprising about 1% to about 5% isopropanol and an ion-pairing agent; (d) applying an organic non-polar mobile phase B to the RP-HPLC column to form a gradient elution, wherein the organic non-polar mobile phase B is a solution comprising about 60% to about 80% isopropanol and about 15% to about 25% acetonitrile and an ion-pairing agent; (e) eluting the cFAE sample; and (f) monitoring the amount of the bispecific antibody formation in the eluted sample.

Also provided are methods of designing a stable multi-specific antibody. The methods comprise (a) performing a manufacturing process to produce a multi-specific antibody, wherein the manufacturing process results in a multi-specific antibody sample comprising an amount of a multi-specific antibody; (b) setting up a reversed-phase high performance liquid chromatography (RP-HPLC) column; (c) injecting the multi-specific antibody sample in a polar aqueous mobile phase A in the RP-HPLC column, wherein the polar aqueous mobile phase A is a solution comprising about 1% to about 5% isopropanol and an ion-pairing agent; (d) applying an organic non-polar mobile phase B to the RP-HPLC column to form a gradient elution, wherein the organic non-polar mobile phase B is a solution comprising about 60% to about 80% isopropanol and about 15% to about 25% acetonitrile and an ion-pairing agent; (e) eluting the multi-specific antibody sample; and (f) monitoring the amount of multi-specific antibody formation in the eluted sample, wherein an increased amount of multi-specific antibody formation as compared to a control indicates the design of a stable multi-specific antibody.

Also provided are methods of obtaining a stability profile of a multi-specific antibody during a manufacturing process to produce a multi-specific antibody. The methods comprise (a) performing a manufacturing process to produce a multi-specific antibody, wherein an amount of a multi-specific antibody sample is obtained at different timepoints during the manufacturing process; (b) setting up a reversed-phase high performance liquid chromatography (RP-HPLC) column; (c) injecting the multi-specific antibody sample from each timepoint in a polar aqueous mobile phase A in the RP-HPLC column, wherein the polar aqueous mobile phase A is a solution comprising about 1% to about 5% isopropanol and an ion-pairing agent; (d) applying an organic non-polar mobile phase B to the RP-HPLC column to form a gradient elution, wherein the organic non-polar mobile phase B is a solution comprising about 60% to about 80% isopropanol and about 15% to about 25% acetonitrile and an ion-pairing agent; (e) eluting the multi-specific antibody sample; and (f) monitoring the amount of multi-specific antibody formation in the eluted sample, wherein the amount of multi-specific antibody formation at each time point provides a stability profile of the multi-specific antibody.

Also provided are methods of obtaining a degraded fragment species profile of a multi-specific antibody during a manufacturing process to produce a multi-specific antibody. The methods comprise (a) performing a manufacturing process to produce a multi-specific antibody, wherein an amount of a multi-specific antibody sample is
obtained at different timepoints during the manufacturing process; (b) setting up a reversed-phase high performance liquid chromatography (RP-HPLC) column; (c) injecting the multispecific antibody sample from each timepoint in a polar aqueous mobile phase A in the RP-HPLC column, wherein the polar aqueous mobile phase A is a solution comprising about 1% to about 5% isopropanol and an ion-pairing agent; (d) applying an organic non-polar mobile phase B to the RP-HPLC column to form a gradient elution, wherein the organic non-polar mobile phase B is a solution comprising about 60% to about 80% isopropanol and about 15% to about 25% acetonitrile and an ion-pairing agent; (e) eluting the multi-specific antibody sample; and (f) monitoring the amount of degraded fragment species in the eluted sample, wherein the amount of degraded fragment species at each time point provides a degraded fragment species profile of the multi-specific antibody manufacturing process.

In embodiments, the polar aqueous mobile phase A is a solution comprising about 1% to about 5% isopropanol. The solution can, for example, comprise about 2% isopropanol.

In certain embodiments, the ion-pairing agent in polar aqueous mobile phase A is selected from the group consisting of trifluoroacetic acid (TFA), difluoroacetic acid (DFA), and formic acid (FA). In certain embodiments, the ion-pairing agent is TFA. The TFA can, for example, be present in the polar aqueous mobile phase A at about 0.1% to about 2%. In certain embodiments, the TFA is present in the polar aqueous mobile phase A at about 0.1%.

In embodiments, the organic non-polar mobile phase is a solution comprising about 60% to about 80% isopropanol and about 15% to about 25% acetonitrile. The solution can, for example, comprise about 70% isopropanol. The solution can, for example, comprise about 20% acetonitrile.

In certain embodiments, the ion-pairing agent in organic non-polar mobile phase B is selected from the group consisting of trifluoroacetic acid (TFA), difluoroacetic acid (DFA), and formic acid (FA). In certain embodiments, the ion-pairing agent is TFA. The TFA can, for example, be present in the organic non-polar mobile phase B at about 0.1% to about 2%. In certain embodiments, the TFA is present in the organic non-polar mobile phase B at about 0.1%.

In certain embodiments, the gradient elution comprises a solution comprising about 75% of polar mobile phase A solution and about 25% of organic non-polar mobile phase B solution at the moment the multi-specific (e.g., bispecific) antibody sample is injected in the RP-HPLC column. The gradient elution can, for example, comprise a solution comprising about 66% of polar mobile phase A solution and about 34% of organic non-polar mobile phase B solution at about 16 minutes after the multi-specific antibody sample is injected in the RP-HPLC column. The gradient elution can, for example, comprise a solution comprising about 5% of polar mobile phase A solution and about 95% of organic non-polar mobile phase B solution at about 20 minutes after the multi-specific antibody sample is injected in the RP-HPLC column. The gradient elution can, for example, comprise a solution comprising about 75% of polar mobile phase A
solution and about 25% of organic non-polar mobile phase B solution at about 21 minutes after the multi-specific antibody sample is injected in the RP-HPLC column.

In certain embodiments, the manufacturing process to produce a multi-specific antibody is selected from the group consisting of a knob in hole process, a strand exchange engineered domain process, a chemical linked bispecific antibody (BsAb) process, an immunoglobulin domain crossover process, and a controlled Fab arm exchange (cFAE) and dual variable domain process.

In certain embodiments, the multi-specific antibody is a bispecific antibody.

### BRIEF DESCRIPTION OF THE DRAWINGS

The foregoing summary, as well as the following detailed description of preferred embodiments of the present application, will be better understood when read in conjunction with the appended drawings. It should be understood, however, that the application is not limited to the precise embodiments shown in the drawings.

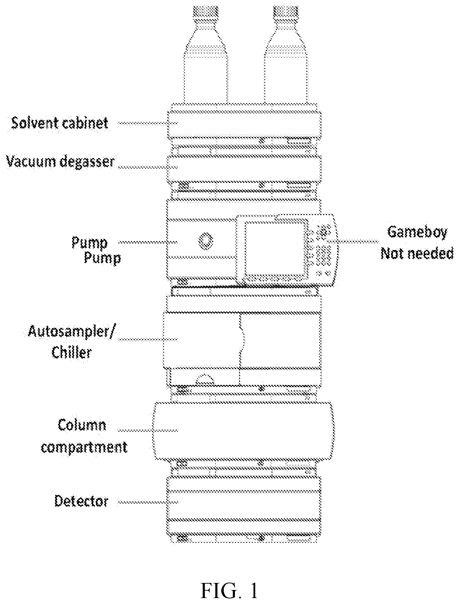

**FIG. 1** shows a schematic of the reversed-phase high performance liquid chromatography (RP-HPLC) single stack configuration.

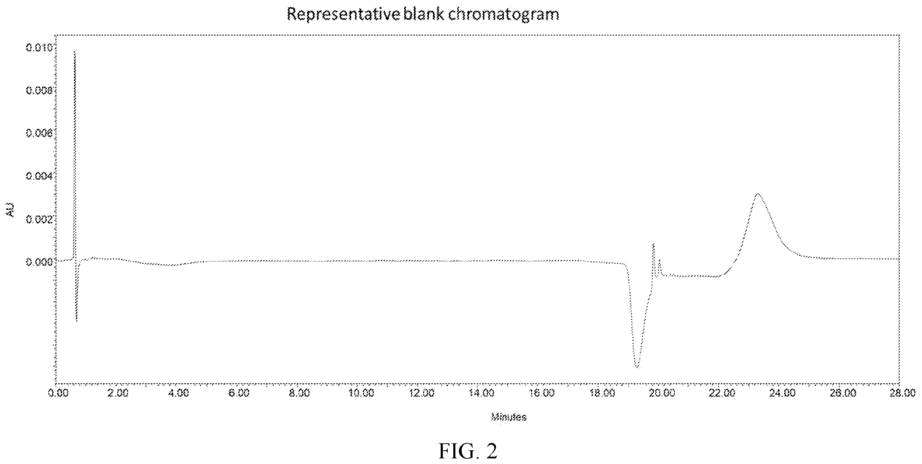

**FIG. 2** shows a graph demonstrating a representative blank chromatogram.
**FIG. 3** shows a graph demonstrating a representative chromatogram of reference material (fully oxidized) for anti-GPRC5D/anti-CD3 bispecific antibody.
**FIG. 4** shows a graph demonstrating a representative chromatogram of partially oxidized anti-GPRC5D/anti-CD3 bispecific antibody.
**FIG. 5** shows a graph demonstrating a representative chromatograms of reference material (fully oxidized) for anti-CD33/anti-CD3 bispecific antibody.
**FIG. 6** shows a graph demonstrating a representative chromatogram of partially oxidized anti-CD33/anti-CD3 bispecific antibody.
**FIG. 7** shows a graph demonstrating a representative chromatogram of reference material (fully oxidized) for anti-TMEFF2/anti-CD3 bispecific antibody.
**FIG. 8** shows a graph demonstrating a representative chromatogram of partially oxidized anti-TMEFF2/anti-CD3 bispecific antibody.
**FIG. 9** shows a graph demonstrating a representative chromatogram of reference material (fully oxidized) for anti-CD123/anti-CD3 bispecific antibody.
**FIG. 10** shows a graph demonstrating a representative chromatogram of partially oxidized anti-CD123/anti-CD3 bispecific antibody.
**FIG. 11** shows a graph demonstrating representative chromatograms of reference material (fully oxidized) for anti-BCMA/anti-CD3 bispecific antibody.
**FIG. 12** shows a graph demonstrating a representative chromatogram of partially oxidized anti-BCMA/anti-CD3 bispecific antibody.

### DETAILED DESCRIPTION OF THE INVENTION

Discussion of documents, acts, materials, devices, articles or the like which has been included in the present specification is for the purpose of providing context for the invention. Such discussion is not an admission that any or all of these matters form part of the prior art with respect to any inventions disclosed or claimed.

Unless defined otherwise, all technical and scientific terms used herein have the same meaning as commonly understood to one of ordinary skill in the art to which this invention pertains. Otherwise, certain terms used herein have the meanings as set forth in the specification.

It must be noted that as used herein and in the appended claims, the singular forms "a," "an," and "the" include plural reference unless the context clearly dictates otherwise.

Unless otherwise stated, any numerical values, such as a concentration or a concentration range described herein, are to be understood as being modified in all instances by the term "about." Thus, a numerical value typically includes ± 10% of the recited value. For example, a concentration of 1 mg/mL includes 0.9 mg/mL to 1.1 mg/mL. Likewise, a concentration range of 1% to 10% (w/v) includes 0.9% (w/v) to 11% (w/v). As used herein, the use of a numerical range expressly includes all possible subranges, all individual numerical values within that range, including integers within such ranges and fractions of the values unless the context clearly indicates otherwise.

Unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the invention.

As used herein, the terms "comprises," "comprising," "includes," "including," "has," "having," "contains" or "containing," or any other variation thereof, will be understood to imply the inclusion of a stated integer or group of integers but not the exclusion of any other integer or group of integers and are intended to be non-exclusive or open-ended. For example, a composition, a mixture, a process, a method, an article, or an apparatus that comprises a list of elements is not necessarily limited to only those elements but can include other elements not expressly listed or inherent to such composition, mixture, process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

As used herein, the conjunctive term "and/or" between multiple recited elements is understood as encompassing both individual and combined options. For instance, where two elements are conjoined by "and/or," a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

As used herein, the term "consists of," or variations such as "consist of" or "consisting of," as used throughout the specification and claims, indicate the inclusion of any recited integer or group of integers, but that no additional integer or group of integers can be added to the specified method, structure, or composition.

As used herein, the term "consists essentially of," or variations such as "consist essentially of" or "consisting essentially of," as used throughout the specification and claims, indicate the inclusion of any recited integer or group of integers, and the optional inclusion of any recited integer or group of integers that do not materially change the basic or novel properties of the specified method, structure or composition. See M.P.E.P. § 2111.03.

It should also be understood that the terms "about," "approximately," "generally," "substantially," and like terms, used herein when referring to a dimension or characteristic of a component of the preferred invention, indicate that the described dimension/characteristic is not a strict boundary or parameter and does not exclude minor variations therefrom that are functionally the same or similar, as would be understood by one having ordinary skill in the art. At a minimum, such references that include a numerical parameter would include variations that, using mathematical and industrial principles accepted in the art (e.g., rounding, measurement or other systematic errors, manufacturing tolerances, etc.), would not vary the least significant digit.

As used herein, the term "polynucleotide," synonymously referred to as "nucleic acid molecule," "nucleotides" or "nucleic acids," refers to any polyribonucleotide or polydeoxyribonucleotide, which can be unmodified RNA or DNA or modified RNA or DNA. "Polynucleotides" include, without limitation single- and double-stranded DNA, DNA that is a mixture of single- and double-stranded regions, single- and double-stranded RNA, and RNA that is mixture of single- and double-stranded regions, hybrid molecules comprising DNA and RNA that can be single-stranded or, more typically, double-stranded or a mixture of single- and double-stranded regions. In addition, "polynucleotide" refers to triple-stranded regions comprising RNA or DNA or both RNA and DNA. The term polynucleotide also includes DNAs or RNAs containing one or more modified bases and DNAs or RNAs with backbones modified for stability or for other reasons. "Modified" bases include, for example, tritylated bases and unusual bases such as inosine. A variety of modifications can be made to DNA and RNA; thus, "polynucleotide" embraces chemically, enzymatically or metabolically modified forms of polynucleotides as typically found in nature, as well as the chemical forms of DNA and RNA characteristic of viruses and cells. "Polynucleotide" also embraces relatively short nucleic acid chains, often referred to as oligonucleotides.

As used herein, the term "vector" is a replicon in which another nucleic acid segment can be operably inserted so as to bring about the replication or expression of the segment.

As used herein, the term "host cell" refers to a cell comprising a nucleic acid molecule of the invention. The "host cell" can be any type of cell, e.g., a primary cell, a cell in culture, or a cell from a cell line. In one embodiment, a "host cell" is a cell transfected with a nucleic acid molecule of the invention. In another embodiment, a "host cell" is a progeny or potential progeny of such a transfected cell. A progeny of a cell may or may not be identical to the parent cell, e.g., due to mutations or environmental influences that can occur in succeeding generations or integration of the nucleic acid molecule into the host cell genome.

The term "expression" as used herein, refers to the biosynthesis of a gene product. The term encompasses the transcription of a gene into RNA. The term also encompasses translation of RNA into one or more polypeptides, and further encompasses all naturally occurring post-transcriptional and post-translational modifications. The expressed bispecific antibody can be within the cytoplasm of a host cell, into the extracellular milieu such as the growth medium of a cell culture or anchored to the cell membrane.

As used herein, the terms "peptide," "polypeptide," or "protein" can refer to a molecule comprised of amino acids and can be recognized as a protein by those of skill in the art. The conventional one-letter or three-letter code for amino acid residues is used herein. The terms "peptide," "polypeptide," and "protein" can be used interchangeably herein to refer to polymers of amino acids of any length. The polymer can be linear or branched, it can comprise modified amino acids, and it can be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, etc.), as well as other modifications known in the art.

The terms "detecting," "detect," or variants thereof, as used herein in the broadest sense include both qualitative and quantitative measurements of a target molecule (e.g., a multi-specific antibody). Detecting includes identifying the mere presence of the target molecule in a sample, as well as determining whether the target molecule is present in the sample at detectable levels.

The term "sample," as used herein, refers to a small portion of a larger quantity of material. Generally, testing according to the methods described herein is performed on a sample. The sample is typically obtained from a multi-specific molecule preparation (e.g., a multi-specific antibody preparation) obtained, for example, from a manufacturing process to produce a multi-specific molecule, as described below.

The terms "inject" or "injecting," as used herein, can refer to loading of sample in an HPLC column in an aqueous phase. The sample can be equilibrated with an equilibration buffer prior to injection and/or loading of the composition to be detected and/or purified.

The terms "elute," "eluting," and/or "elution," as used herein can refer to the removal of a product (e.g., a multi-specific antibody) from a chromatography material. Elution buffer is the buffer used to elute the multi-specific antibody from a chromatography material.

### Systems, Materials, and Methods for detecting multi-specific molecule formation

Embodiments of the present invention are set out in the enclosed claims.

In one embodiment, the present invention provides a method of detecting formation of a multi-specific molecule, the method comprising:
a. obtaining a multi-specific molecule sample;
b. obtaining a reversed-phase high performance liquid chromatography (RP-HPLC) column;
c. contacting the multi-specific molecule sample in a polar aqueous mobile phase A with the RP-HPLC column, wherein the polar aqueous mobile phase A is a solution comprising about 1% to about 5% isopropanol and an ion-pairing agent;
d. contacting an organic non-polar mobile phase B with the RP-HPLC column, wherein the organic non-polar mobile phase B is a solution comprising about 60% to about 80% isopropanol and about 15% to about 25% acetonitrile and an ion-pairing agent;
e. eluting the multi-specific molecule sample; and
f. monitoring the amount of multi-specific molecule formation in the eluted multispecific molecule sample.

In one embodiment, the present invention provides a system comprising a reversed-phase high performance liquid chromatography (RP-HPLC) member for detecting formation of a multi-specific molecule, the system comprising:
a. a multi-specific molecule sample in a polar aqueous mobile phase A, wherein the polar aqueous mobile phase A is a solution comprising about 1% to about 5% isopropanol and an ion pairing agent;
b. a RP-HPLC column;
c. an organic non-polar mobile phase B, wherein the organic non-polar mobile phase B is a solution comprising about 60% to about 80% isopropanol and about 15% to about 25% acetonitrile and an ion pairing agent;

wherein the RP-HPLC column is configured to be contacted with the multi-specific molecule sample in polar aqueous mobile phase A, and
wherein the RP-HPLC is further configured to be contacted with the organic non-polar mobile phase B to elute a multi-specific molecule sample, wherein an amount of multispecific molecule formation can be identified in the eluted multi-specific molecule sample.

In certain embodiments, the multi-specific molecule is a multi-specific antibody. In certain embodiments, the multi-specific antibody is a bispecific antibody.

Provided herein are methods of detecting formation of a multi-specific antibody while performing a manufacturing process to produce a multi-specific antibody. The methods comprise (a) performing a manufacturing process to produce a multi-specific antibody, wherein the manufacturing process results in a multi-specific antibody sample comprising an amount of a multi-specific antibody; (b) setting up a reversed-phase high performance liquid chromatography (RP-HPLC) column; (c) injecting the multi-specific antibody sample in a polar aqueous mobile phase A in the RP-HPLC column, wherein the polar aqueous mobile phase A is a solution comprising about 1% to about 5% isopropanol and an ion-pairing agent; (d) applying an organic non-polar mobile phase B to the RP-HPLC column to form a gradient elution, wherein the organic non-polar mobile phase B is a solution comprising about 60% to about 80% isopropanol and about 15% to about 25% acetonitrile and an ion-pairing agent; (e) eluting the multi-specific antibody sample; and (f) monitoring the amount of multi-specific antibody formation in the eluted sample.

Also provided herein are methods of detecting formation of a bispecific antibody during a controlled FAB arm exchange (cFAE) to produce a bispecific antibody. The methods comprise (a) performing a controlled FAB arm exchange (cFAE) to produce a cFAE sample, wherein the cFAE sample comprises an amount of a bispecific antibody; (b) setting up a reversed-phase high performance liquid chromatography (RP-HPLC) column; (c) injecting the cFAE sample in a polar aqueous mobile phase A in the RP-HPLC column, wherein the polar aqueous mobile phase A is a solution comprising about 1% to about 5% isopropanol and an ion-pairing agent; (d) applying an organic non-polar mobile phase B to the RP-HPLC column to form a gradient elution, wherein the organic non-polar mobile phase B is a solution comprising about 60% to about 80% isopropanol and about 15% to about 25% acetonitrile and an ion-pairing agent; (e) eluting the cFAE sample; and (f) monitoring the amount of the bispecific antibody formation in the eluted sample.

In certain embodiments, the amount of the multi-specific antibody formed can be determined by preparing and testing a reference sample. The amount of partially formed multi-specific antibody can be determined by comparing to the reference sample (fully formed). The reference sample can comprise a sample with 80-100% of fully formed multispecific antibody. Comparing the process manufacturing samples to the reference samples can provide a determination of the efficiency and effectiveness of the manufacturing process to produce the multi-specific antibody by being able to determine both the amount of the fully formed multi-specific antibody and its partially formed antibody fragments.

Also provided are methods of utilizing this assay in process development kinetic studies to develop a multi-specific antibody. The methods comprise (a) performing a research and development process to produce a multi-specific antibody, wherein the research and development process results in a multi-specific antibody sample comprising an amount of a multi-specific antibody; (b) setting up a reversed-phase high performance liquid chromatography (RP-HPLC) column; (c) injecting the multi-specific antibody sample in a polar aqueous mobile phase A in the RP-HPLC column, wherein the polar aqueous mobile phase A is a solution comprising about 1% to about 5% isopropanol and an ion-pairing agent; (d) applying an organic non-polar mobile phase B to the RP-HPLC column to form a gradient elution, wherein the organic non-polar mobile phase B is a solution comprising about 60% to about 80% isopropanol and about 15% to about 25% acetonitrile and an ion-pairing agent; (e) eluting the multi-specific antibody sample; and (f) monitoring the amount of multi-specific antibody formation in the eluted sample, wherein an increased amount of multi-specific antibody formation as compared to a reference sample indicates the successful design of a multi-specific antibody.

Also provided are methods of obtaining a stability profile of a multi-specific antibody during a manufacturing process to produce a multi-specific antibody. The methods comprise (a) performing a manufacturing process to produce a multi-specific antibody, wherein an amount of a multi-specific antibody sample is obtained at different timepoints during the manufacturing process; (b) setting up a reversed-phase high performance liquid chromatography (RP-HPLC) column; (c) injecting the multi-specific antibody sample from each timepoint in a polar aqueous mobile phase A in the RP-HPLC column, wherein the polar aqueous mobile phase A is a solution comprising about 1% to about 5% isopropanol and an ion-pairing agent; (d) applying an organic non-polar mobile phase B to the RP-HPLC column to form a gradient elution, wherein the organic non-polar mobile phase B is a solution comprising about 60% to about 80% isopropanol and about 15% to about 25% acetonitrile and an ion-pairing agent; (e) eluting the multi-specific antibody sample; and (f) monitoring the amount of multi-specific antibody formation in the eluted sample, wherein the amount of multi-specific antibody formation at each time point provides a stability profile of the multi-specific antibody.

Comparing the process manufacturing samples to the reference samples can provide a determination of the efficiency and effectiveness of the manufacturing process to produce the multi-specific antibody by being able to determine both the amount of the
fully formed multi-specific antibody and its partially formed antibody fragments. An increase in the amount of fully formed multi-specific antibody as compared to the reference samples can indicate that the design of the multi-specific antibody is favorable to produce a multi-specific antibody that does not comprise unformed and/or partially formed multispecific antibody fragments.

As disclosed herein, reversed-phase high pressure liquid chromatography (RP-HPLC) is a chromatography technique using a hydrophobic stationary phase (e.g., a stationary phase comprising alkyl chains covalently bonded to the stationary phase particles). The use of the hydrophobic stationary phase is the reverse of normal phase chromatography, as the polarity of the mobile and stationary phases are inverted. RP-HPLC employs a polar aqueous mobile phase. As a result, hydrophobic molecules in the polar mobile phase tend to adsorb to the hydrophobic stationary phase, and hydrophilic molecules in the mobile phase will pass through the column and are eluted first. Hydrophobic molecules can be eluted from the column by decreasing the polarity of the mobile phase using an organic (non-polar) solvent, which reduces the hydrophobic interactions. The more hydrophobic the molecule, the more strongly it binds the stationary phase, and the higher the concentration of the organic solvent that is required to elute the molecule.

In embodiments, the polar aqueous mobile phase is a solution comprising about 1% to about 5% isopropanol. The solution can, for example, comprise isopropanol at about 1%, about 2%, about 3%, about 4%, about 5%, or any value in between. In preferred embodiments, the polar aqueous mobile phase comprises about 2% isopropanol.

In certain embodiments, the ion-pairing agent in mobile phase A is selected from the group consisting of trifluoroacetic acid (TFA), difluoroacetic acid (DFA), and formic acid (FA).

In certain embodiments, the ion-pairing agent is TFA. The TFA can, for example, be present in the polar aqueous mobile phase at about 0.1% to about 2%. The TFA can, for example, be present in the polar aqueous mobile phase at about 0.1%, about 0.2%, about 0.3%, about 0.4%, about 0.5%, about 0.6%, about 0.7%, about 0.8%, about 0.9%, about 1.0%, about 1.1%, about 1.2%, about 1.3%, about 1.4%, about 1.5%, about 1.6%, about 1.7%, about 1.8%, about 1.9%, about 2.0% or any value in between. In preferred embodiments, the TFA is present in the polar aqueous mobile phase at about 0.1%.

In certain embodiments, the ion-pairing agent is DFA. The DFA can, for example, be present in the polar aqueous mobile phase at about 0.1% to about 2%. The DFA can, for example, be present in the polar aqueous mobile phase at about 0.1%, about
0.2%, about 0.3%, about 0.4%, about 0.5%, about 0.6%, about 0.7%, about 0.8%, about 0.9%, about 1.0%, about 1.1%, about 1.2%, about 1.3%, about 1.4%, about 1.5%, about 1.6%, about 1.7%, about 1.8%, about 1.9%, about 2.0% or any value in between. In preferred embodiments, the DFA is present in the polar aqueous mobile phase at about 0.1%.

In certain embodiments, the ion-pairing agent is FA. The FA can, for example, be present in the polar aqueous mobile phase at about 0.5% to about 5%. The FA can, for example, be present in the polar aqueous mobile phase at about 0.5%, about 0.6%, about 0.7%, about 0.8%, about 0.9%, about 1.0%, about 1.1%, about 1.2%, about 1.3%, about 1.4%, about 1.5%, about 1.6%, about 1.7%, about 1.8%, about 1.9%, about 2.0%, about 2.1%, about 2.2%, about 2.3%, about 2.4%, about 2.5%, about 2.6%, about 2.7%, about 2.8%, about 2.9%, about 3.0%, about 3.1%, about 3.2%, about 3.3%, about 3.4%, about 3.5%, about 3.6%, about 3.7%, about 3.8%, about 3.9%, about 4.0%, about 4.1%, about 4.2%, about 4.3%, about 4.4%, about 4.5%, about 4.6%, about 4.7%, about 4.8%, about 4.9%, about 5.0%, or any value in between.

The organic non-polar mobile phase solution can, for example, comprise isopropanol at about 60%, about 65%, about 70%, about 75%, about 80% or any value in between. In preferred embodiments, the organic non-polar mobile phase solution comprises about 70% isopropanol. The organic non-polar mobile phase solution can, for example, comprise acetonitrile at about 15%, about 16%, about 17%, about 18%, about 19%, about 20%, about 21%, about 22%, about 23%, about 24%, about 25%, or any value in between. In preferred embodiments, the organic non- polar mobile phase comprises about 20% acetonitrile.

In certain embodiments, the organic non-polar mobile phase comprises about 70% isopropanol, about 20% acetonitrile, and about 10% water; about 80% isopropanol and about 20% acetonitrile; and about 75% isopropanol and about 25% acetonitrile.

In certain embodiments, the ion-pairing agent in mobile phase B is selected from the group consisting of trifluoroacetic acid (TFA), difluoroacetic acid (DFA), and formic acid (FA).

In certain embodiments, the ion-pairing agent is TFA. The TFA can, for example, be present in the polar aqueous mobile phase at about 0.1% to about 2%. The TFA can, for example, be present in the polar aqueous mobile phase at about 0.1%, about 0.2%, about 0.3%, about 0.4%, about 0.5%, about 0.6%, about 0.7%, about 0.8%, about 0.9%, about 1.0%, about 1.1%, about 1.2%, about 1.3%, about 1.4%, about 1.5%, about 1.6%, about 1.7%, about 1.8%, about 1.9%, about 2.0% or any value in between. In preferred embodiments, the TFA is present in the polar aqueous mobile phase at about 0.1%.

In certain embodiments, the ion-pairing agent is DFA. The DFA can, for example, be present in the polar aqueous mobile phase at about 0.1% to about 2%. The DFA can, for example, be present in the polar aqueous mobile phase at about 0.1%, about 0.2%, about 0.3%, about 0.4%, about 0.5%, about 0.6%, about 0.7%, about 0.8%, about 0.9%, about 1.0%, about 1.1%, about 1.2%, about 1.3%, about 1.4%, about 1.5%, about 1.6%, about 1.7%, about 1.8%, about 1.9%, about 2.0% or any value in between. In preferred embodiments, the DFA is present in the polar aqueous mobile phase at about 0.1%.

In certain embodiments, the ion-pairing agent is FA. The FA can, for example, be present in the polar aqueous mobile phase at about 1.0% to about 5%. The FA can, for example, be present in the polar aqueous mobile phase at about 1.0%, about 1.1%, about 1.2%, about 1.3%, about 1.4%, about 1.5%, about 1.6%, about 1.7%, about 1.8%, about 1.9%, about 2.0%, about 2.1%, about 2.2%, about 2.3%, about 2.4%, about 2.5%, about 2.6%, about 2.7%, about 2.8%, about 2.9%, about 3.0%, about 3.1%, about 3.2%, about 3.3%, about 3.4%, about 3.5%, about 3.6%, about 3.7%, about 3.8%, about 3.9%, about 4.0%, about 4.1%, about 4.2%, about 4.3%, about 4.4%, about 4.5%, about 4.6%, about 4.7%, about 4.8%, about 4.9%, about 5.0%, or any value in between.

In certain embodiments, the gradient elution comprises a solution comprising about 75% of polar mobile phase A solution and about 25% of organic non-polar mobile phase B solution at the moment the multi-specific antibody sample is injected in the RP-HPLC column. The gradient elution can, for example, comprise a solution comprising about 66% of polar mobile phase A solution and about 34% of organic non-polar mobile phase B solution at about 16 minutes after the multi-specific antibody sample is injected in the RP-HPLC column. The gradient elution can, for example, comprise a solution comprising about 5% of polar mobile phase A solution and about 95% of organic non-polar mobile phase B solution at about 20 minutes after the multi-specific antibody sample is injected in the RP-HPLC column. The gradient elution can, for example, comprise a solution comprising about 75% of polar mobile phase A solution and about 25% of organic non-polar mobile phase B solution at about 21 minutes after the multispecific antibody sample is injected in the RP-HPLC column.

### Antibodies

Provided herein are methods of detecting multi-specific antibody formation while performing a manufacturing process to produce a multi-specific antibody, methods of designing a stable multi-specific antibody, and methods of obtaining a stability profile or obtaining a degraded fragment species profile during a manufacturing process to produce a multi-specific antibody. In certain embodiments, the multi-specific antibody is a bispecific antibody.

As used herein, the term "antibody" is used in a broad sense and includes immunoglobulin or antibody molecules including human, humanized, composite and chimeric antibodies and antibody fragments that are monoclonal or polyclonal. In general, antibodies are proteins or peptide chains that exhibit binding specificity to a specific antigen. Antibody structures are well known. Immunoglobulins can be assigned to five major classes (i.e., IgA, IgD, IgE, IgG and IgM), depending on the heavy chain constant domain amino acid sequence. IgA and IgG are further sub-classified as the isotypes IgA1, IgA2, IgG1, IgG2, IgG3 and IgG4. Accordingly, the antibodies of the invention can be of any of the five major classes or corresponding sub-classes. Preferably, the antibodies of the invention are IgG1, IgG2, IgG3 or IgG4. Antibody light chains of vertebrate species can be assigned to one of two clearly distinct types, namely kappa and lambda, based on the amino acid sequences of their constant domains. Accordingly, the antibodies of the invention can contain a kappa or lambda light chain constant domain. According to particular embodiments, the antibodies of the invention include heavy and/or light chain constant regions from rat or human antibodies. In addition to the heavy and light constant domains, antibodies contain an antigen-binding region that is made up of a light chain variable region and a heavy chain variable region, each of which contains three domains (i.e., complementarity determining regions 1-3; CDR1, CDR2, and CDR3). The light chain variable region domains are alternatively referred to as LCDR1, LCDR2, and LCDR3, and the heavy chain variable region domains are alternatively referred to as HCDR1, HCDR2, and HCDR3.

As used herein, the term an "isolated antibody" refers to an antibody which is substantially free of other antibodies having different antigenic specificities (e.g., an isolated multi-specific antibody that specifically bind two or more antigens is substantially free of multi-specific antibodies that do not bind the two or more antigens). In addition, an isolated antibody is substantially free of other cellular material and/or chemicals.

As used herein, the term "monoclonal antibody" refers to an antibody obtained from a population of substantially homogeneous antibodies, i.e., the individual antibodies comprising the population are identical except for possible naturally occurring mutations that may be present in minor amounts. The monoclonal antibodies of the invention can be made by the hybridoma method, phage display technology, single lymphocyte gene cloning technology, or by recombinant DNA methods. For example, the monoclonal antibodies can be produced by a hybridoma which includes a B cell obtained from a transgenic nonhuman animal, such as a transgenic mouse or rat, having a genome comprising a human heavy chain transgene and a light chain transgene.

As used herein, the term "antigen-binding fragment" refers to an antibody fragment such as, for example, a diabody, a Fab, a Fab', a F(ab')2, an Fv fragment, a disulfide stabilized Fv fragment (dsFv), a (dsFv)₂, a bispecific dsFv (dsFv-dsFv'), a disulfide stabilized diabody (ds diabody), a single-chain antibody molecule (scFv), a single domain antibody (sdab) an scFv dimer (bivalent diabody), a multi-specific antibody formed from a portion of an antibody comprising one or more CDRs, a camelized single domain antibody, a nanobody, a domain antibody, a bivalent domain antibody, or any other antibody fragment that binds to an antigen but does not comprise a complete antibody structure. An antigen-binding fragment is capable of binding to the same antigen to which the parent antibody or a parent antibody fragment binds. According to particular embodiments, the antigen-binding fragment comprises a light chain variable region, a light chain constant region, and an Fd segment of the heavy chain. According to other particular embodiments, the antigen-binding fragment comprises Fab and F(ab').

As used herein, the term "single-chain antibody" refers to a conventional single-chain antibody in the field, which comprises a heavy chain variable region and a light chain variable region connected by a short peptide of about 15 to about 20 amino acids. As used herein, the term "single domain antibody" refers to a conventional single domain antibody in the field, which comprises a heavy chain variable region and a heavy chain constant region or which comprises only a heavy chain variable region.

As used herein, the term "human antibody" refers to an antibody produced by a human or an antibody having an amino acid sequence corresponding to an antibody produced by a human made using any technique known in the art. This definition of a human antibody includes intact or full-length antibodies, fragments thereof, and/or antibodies comprising at least one human heavy and/or light chain polypeptide.

As used herein, the term "humanized antibody" refers to a non-human antibody that is modified to increase the sequence homology to that of a human antibody, such that the antigen-binding properties of the antibody are retained, but its antigenicity in the human body is reduced.

As used herein, the term "chimeric antibody" refers to an antibody wherein the amino acid sequence of the immunoglobulin molecule is derived from two or more species. The variable region of both the light and heavy chains often corresponds to the variable region of an antibody derived from one species of mammal (e.g., mouse, rat, rabbit, etc.) having the desired specificity, affinity, and capability, while the constant regions correspond to the sequences of an antibody derived from another species of mammal (e.g., human) to avoid eliciting an immune response in that species.

As used herein, the term "multi-specific antibody" refers to an antibody that comprises a plurality of immunoglobulin variable domain sequences, wherein a first immunoglobulin variable domain sequence of the plurality has binding specificity for a first epitope and a second immunoglobulin variable domain sequence of the plurality has binding specificity for a second epitope. In an embodiment, the first and second epitopes are on the same antigen, e.g., the same protein (or subunit of a multimeric protein). In an embodiment, the first and second epitopes overlap or substantially overlap. In an embodiment, the first and second epitopes do not overlap or do not substantially overlap. In an embodiment, the first and second epitopes are on different antigens, e.g., the different proteins (or different subunits of a multimeric protein). In an embodiment, a multispecific antibody comprises a third, fourth, or fifth immunoglobulin variable domain. In an embodiment, a multispecific antibody is a bispecific antibody molecule, a trispecific antibody molecule, or a tetraspecific antibody molecule.

As used herein, the term "bispecifc antibody" refers to a multi-specific antibody that binds no more than two epitopes or two antigens. A bispecific antibody is characterized by a first immunoglobulin variable domain sequence which has binding specificity for a first epitope and a second immunoglobulin variable domain sequence that has binding specificity for a second epitope. In an embodiment, the first and second epitopes are on the same antigen, e.g., the same protein (or subunit of a multimeric protein). In an embodiment, the first and second epitopes overlap or substantially overlap. In an embodiment, the first and second epitopes are on different antigens, e.g., the different proteins (or different subunits of a multimeric protein). In an embodiment, a bispecific antibody comprises a heavy chain variable domain sequence and a light chain variable domain sequence which have binding specificity for a first epitope and a heavy chain variable domain sequence and a light chain variable domain sequence which have binding specificity for a second epitope. In an embodiment, a bispecific antibody comprises a half antibody, or fragment thereof, having binding specificity for a first epitope and a half antibody, or fragment thereof, having binding specificity for a second epitope. In an embodiment, a bispecific antibody comprises a scFv, or fragment thereof, having binding specificity for a first epitope, and a scFv, or fragment thereof, having binding specificity for a second epitope.

Provided herein are methods of detecting multi-specific antibody formation after performing a manufacturing process to produce a multi-specific antibody. Also provided are methods of obtaining a stability profile of a multi-specific antibody and methods of obtaining a degraded fragment species profile during a manufacturing process to produce a multi-specific antibody. In certain embodiments, the manufacturing process to produce a multi-specific antibody is selected from the group consisting of a controlled arm exchange (cFAE) process, a knob in hole process, a heavy chain heterodimerization process, and an *in vitro* disulfide bond isomerization process.

Full length bispecific antibodies of the invention can be generated for example using Fab arm exchange (or half molecule exchange) between two mono specific bivalent antibodies by introducing substitutions at the heavy chain CH3 interface in each half molecule to favor heterodimer formation of two antibody half molecules having distinct specificity either *in vitro* in cell-free environment or using co-expression. The Fab arm exchange reaction is the result of a disulfide-bond isomerization reaction and dissociation-association of CH3 domains. The heavy-chain disulfide bonds in the hinge regions of the parent mono specific antibodies are reduced. The resulting free cysteines of one of the parent monospecific antibodies form an inter heavy-chain disulfide bond with cysteine residues of a second parent monospecific antibody molecule and simultaneously CH3 domains of the parent antibodies release and reform by dissociation-association. The CH3 domains of the Fab arms can be engineered to favor heterodimerization over homodimerization. The resulting product is a bispecific antibody having two Fab arms or half molecules which each binding a distinct epitope, i.e. an epitope on a first antigen and an epitope on a second antigen.

"Homodimerization" as used herein refers to an interaction of two heavy chains having identical CH3 amino acid sequences. "Homodimer" as used herein refers to an antibody having two heavy chains with identical CH3 amino acid sequences.

"Heterodimerization" as used herein refers to an interaction of two heavy chains having non-identical CH3 amino acid sequences. "Heterodimer" as used herein refers to an antibody having two heavy chains with non-identical CH3 amino acid sequences.

The "knob-in-hole" strategy (see, e.g., PCT Publ. No. WO2006/028936) can be used to generate full length bispecific antibodies. Briefly, selected amino acids forming the interface of the CH3 domains in human IgG can be mutated at positions affecting CH3 domain interactions to promote heterodimer formation. An amino acid with a small side chain (hole) is introduced into a heavy chain of an antibody specifically binding a first antigen and an amino acid with a large side chain (knob) is introduced into a heavy chain of an antibody specifically binding a second antigen. After co-expression of the two antibodies, a heterodimer is formed as a result of the preferential interaction of the heavy chain with a "hole" with the heavy chain with a "knob." Exemplary CH3 substitution pairs forming a knob and a hole are (expressed as modified position in the first CH3 domain of the first heavy chain/modified position in the second CH3 domain of the second heavy chain): T366Y/F405A, T366W/ F405W, F405W/Y407A, T394W/Y407T, T394S/Y407A, T366W/T394S, F405W/T394S and T366W/T366S_L368A_Y407V.

Other strategies such as promoting heavy chain heterodimerization using electrostatic interactions by substituting positively charged residues at one CH3 surface and negatively charged residues at a second CH3 surface can be used, as described in US Pat. Publ. No. US2010/0015133; US Pat. Publ. No. US2009/0182127; US Pat. Publ. No. US2010/028637; or US Pat. Publ. No. US2011/0123532. In other strategies, heterodimerization can be promoted by the following substitutions (expressed as modified position in the first CH3 domain of the first heavy chain/modified position in the second CH3 domain of the second heavy chain): L351Y_F405AY407V/T394W, T366I_K392M_T394W/F405A_Y407V, T366L_K392M_T394W/F405A_Y407V, L351Y_Y407A/T366A_K409F, L351Y_Y407A/T366V K409F Y407A/T366A_K409F, or T350V_L351Y_F405A Y407V/T350V_T366L_K392L_T394W as described in U.S. Pat. Publ. No. US2012/0149876 or U.S. Pat. Publ. No. US2013/0195849.

In addition to methods described above, bispecific antibodies of the invention can be generated *in vitro* in a cell-free environment by introducing asymmetrical mutations in the CH3 regions of two mono specific homodimeric antibodies and forming the bispecific heterodimeric antibody from two parent monospecific homodimeric antibodies in reducing conditions to allow disulfide bond isomerization according to methods described in PCT Pat. Publ. No. WO2011/131746. In the methods, the first monospecific bivalent antibody (e.g., anti-CD33 antibody) and the second monospecific bivalent antibody (e.g., anti-CD3 antibody) are engineered to have certain substitutions at the CH3 domain that promotes heterodimer stability; the antibodies are incubated together under reducing conditions sufficient to allow the cysteines in the hinge region to undergo disulfide bond isomerization; thereby generating the bispecific antibody by Fab arm exchange. The incubation conditions can optionally be restored to non-reducing conditions. Exemplary reducing agents that can be used are 2-mercaptoethylamine (2-MEA), dithiothreitol (DTT), dithioerythritol (DTE), glutathione, tris (2-carboxyethyl) phosphine (TCEP), L-cysteine and beta-mercaptoethanol, preferably a reducing agent selected from the group consisting of: 2-mercaptoethylamine, dithiothreitol and tris (2-carboxyethyl) phosphine. For example, incubation for at least 90 min at a temperature of at least 20°C in the presence of at least 25 mM 2-MEA or in the presence of at least 0.5 mM dithiothreitol at a pH from 5-8, for example at pH of 7.0 or at pH of 7.4 can be used.

### EXAMPLES

### Example 1: Reversed-phase high performance liquid chromatography for determining bispecific antibody formation after controlled FAB arm exchange (FAE).

Reversed-phase high performance liquid chromatography (RP-HPLC) separates molecules based on a hydrophobic absorption/desorption process across a hydrophobic solid support. Using an increasing polar mobile phase, the hydrophobic binding interaction between the protein and the solid support resulted in desorption of the analyte from the column. In this particular method, where antibody formation is established through reoxidation of parental mAbs, RP-HPLC was employed to quantitate intact, fully oxidized bispecific DuoBody^{®} (anti-GPRC5D/anti-CD3, anti-CD33/anti-CD3, anti-TMEFF2/anti-CD3, anti-7959/anti-CD3, anti-BCMA/anti-CD3) from partially reoxidized fragments. Test articles were sampled after controlled FAB arm exchange (FAE) and injected onto the column in a highly polar aqueous mobile phase containing an ion pairing agent (trifluoroacetic acid) used for improving peak shape and molecule retention. An increasing organic non-polar mobile phase, also containing trifluoracetic acid, was then applied to the column in a gradient elution to elute the molecules based on their hydrophobic properties. The column eluate was monitored continuously at 280 nm and the relative amount of each IgG was measured by comparing peak area count to the sub fragment IgG species.

### Example 2: Preparation of Reagents/Solutions for bispecific antibodies

Note A: Different total volumes of mobile phase A and mobile phase B can be prepared, but the ratios of each component must be maintained.

Note B: Trifluoroacetic acid (TFA) has a low boiling point and high volatility. It is recommended to store TFA at +2-8°C for dispensing accurately by pipetting.

**Table 1: Mobile Phase A (98% Water/2% Isopropanol (IPA)/0.1% TFA)**

| **Component** | **Amount** |
|---|---|
| Deionized Water | 979 mL |
| Isopropanol | 20 mL |
| TFA | 1 mL |

| **Expiration (Room Temperature)** | |
|---|---|
| 1 week | |

To prepare mobile phase A, in a fume hood, approximately 800 mL of deionized water was added to a 1 liter beaker. In a 50 mL graduated cylinder, 20 mL of isopropanol was added. The 20 mL of isopropanol was added to the 1 liter beaker and the deionized water and isopropanol was mixed with a stir bar for 3 minutes. 1 mL of refrigerated TFA was carefully pipetted and added to the 1 liter beaker containing isopropanol and deionized water. The TFA was delivered below the solution surface, and the mixture was stirred for an additional 3 minutes. (Note: delivery of the TFA below the solution surface was critical for assay retention times due to the high volitalization of TFA). The solution from the 1 liter beaker was transferred to a 1 liter graduated cylinder and was filled to the 1 liter mark with deionized water. The solution was then transferred to mobile phase A bottle.

**Table 2: Mobile Phase B (70% IPA, 20% acetonitrile, 9.9% water, 0.1% TFA)**

| **Component** | **Amount** |
|---|---|
| Isopropanol | 700 mL |
| Acetonitrile | 200 mL |
| Deionized Water | 99 mL |
| TFA | 1 mL |

| **Expiration (Room Temperature)** | |
|---|---|
| 1 week | |

To prepare mobile phase B, in a fume hood, approximately 99 mL of deionized water was added to a 1 liter beaker followed by 200 mL of acetonitrile. The solution was mixed together for 3 minutes with a stir bar. 1 mL of refrigerated TFA was carefully pipetted and added to the 1 liter beaker containing acetonitrile and deionized water. The TFA was delivered below the solution surface, and the mixture was stirred for an additional 3 minutes. (Note: delivery of the TFA below the solution surface was critical for assay retention times due to the high volitalization of TFA). The solution was transferred from the 1 liter beaker to a 1 liter graduated cylinder and filled to the 1 liter mark with isopropanol. The solution was then transferred to mobile phase B bottle.

### Example 3: Sample and standard preparations

The bispecific antibody is made from reducing the parental homodimer bispecifics and reoxidizing these reactants via a cFAE to produce the heterodimer bispecific antibody. To prepare a post cFAE bispecific antibody process sample for testing, the process sample is diluted from the cFAE UF/DF concentration, determined by A₂₈₀, to a final testing concentration of 2 mg/mL (diluted in mobile phase A) and according to Table 3.

**Table 3: Example dilution for anti-CPRC5D/anti-CD3 bispecific antibody sample (2 mg/mL)**

| **Stock Conc. (mg/mL)** | **Sample Volume (µL)** | **Diluent Volume (µL)** | **Total Volume (µL)** | **Target Conc. (mg/mL)** |
|---|---|---|---|---|
| 35.0 | 50.0 | 825 | 875 | 2.0 |

To prepare a reference material sample at 2 mg/mL, a stock concentration of the bispecific antibody was diluted in mobile phase A as the sample diluent according to Table 4.

**Table 4: Example of a reference sample dilution for anti-CPRC5D/anti-CD3 bispecific antibody**

| **Stock Conc. (mg/mL)** | **Sample Volume (µL)** | **Diluent Volume (µL)** | **Total Volume (µL)** | **Target Conc. (mg/mL)** |
|---|---|---|---|---|
| 10.2 | 78.4 | 321.6 | 400 | 2.0 |

### Example 4: HPLC instrument setup

The instrument parameters are provided in Table 5 and Table 6. The column module thermostat was controlled at 80°C to heat both the right and left side compartments. The connecting thermostat capillary tubing was plumbed to the left side (3 µL) heating element. To avoid over-pressurization of the LC system and damage to the column, the thermostat temperature of 80°C was reached before any flow was started to the system.

**Table 5: Bispecific instrument parameters (Universal)**

| **Parameter** | **Value** |
|---|---|
| Flow rate | 0.5 mL/min |
| Injection Volume | 5 µL |
| Column Temperature | 80°C |
| Detector Wavelength | 280 nm |
| Run Time | 28 minutes |
| Post Run Time | None |
| Auto-sample temperature | 4°C |
| Maximum Pressure | 400 Bar |

**Table 6: Instrument parameters (Agilent 1200 or 1260)**

| **Parameters** | **Agilent 1200-1260** |
|---|---|
| Bandwidth | 4 |
| Slit | 4 nm |
| Reference wavelength, Bandwidth | 360, 100 nm |
| Peak Width | > 0.1 min |

**Table 7: Example gradient for anti-CPRC5D/anti-CD3 bispecific antibody**

| **Time (min)** | **%A** | **%B** |
|---|---|---|
| 0.0 | 75 | 25 |
| 2 | 70 | 30 |
| 16 | 66 | 34 |
| 17 | 66 | 34 |
| 17.1 | 5 | 95 |
| 20 | 5 | 95 |
| 21 | 75 | 25 |
| 28 | 75 | 25 |

**Table 8: Example sample sequence for anti-CPRC5D/anti-CD3 bispecific antibody**

| **Injection Type** | **No. of injections** |
|---|---|
| Column condition (mobile phase A) | 1 |
| Anti-GPRC5D/anti-CD3 reference material | 2 |
| FAE in-process sample | 1-10 |
| Anti-GPRC5D/anti-CD3 reference material | 2 |
| Blank (mobile phase A) | 1 |

| | |
|---|---|
| Note: The sequence can be extended for multiple samples. | |

### Example 5: Integrating results

Note: Manual integration was used and integrated with a processing method.

### Peak Integrations for Reference Material.

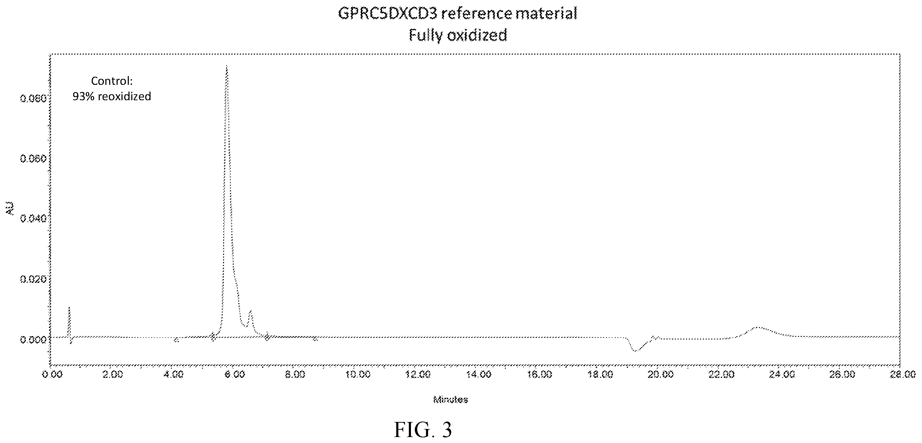

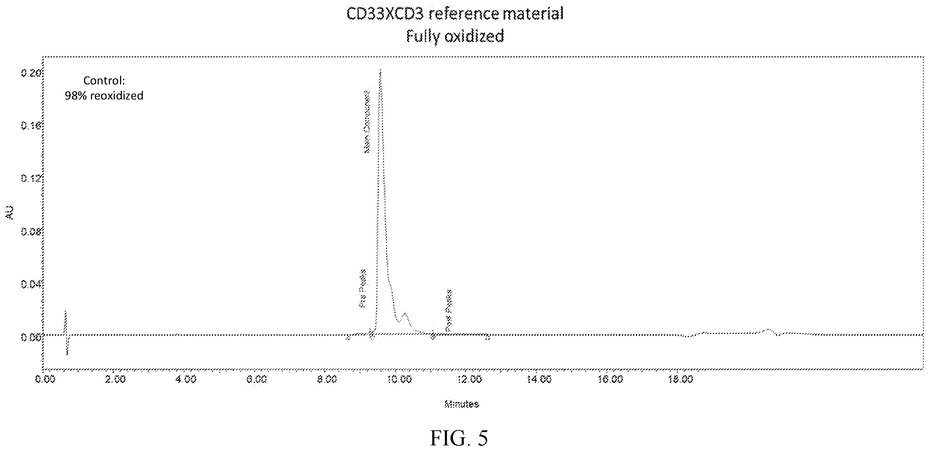

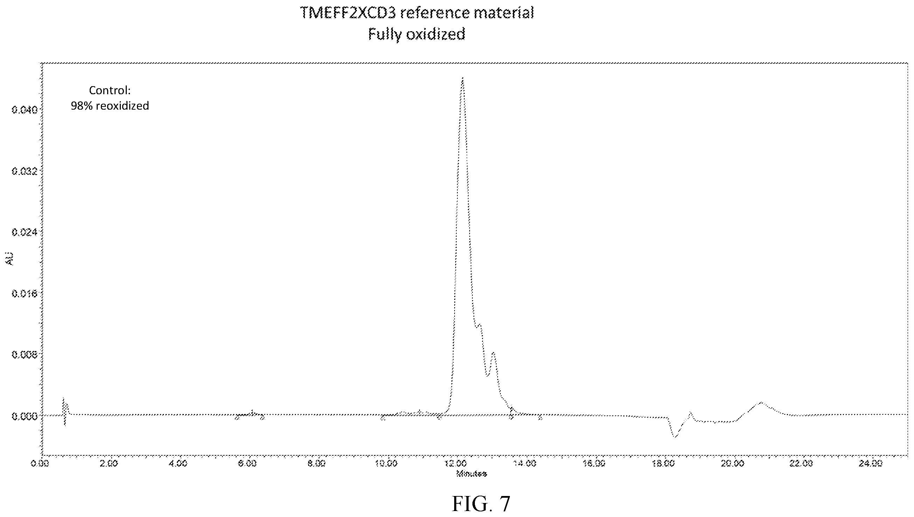

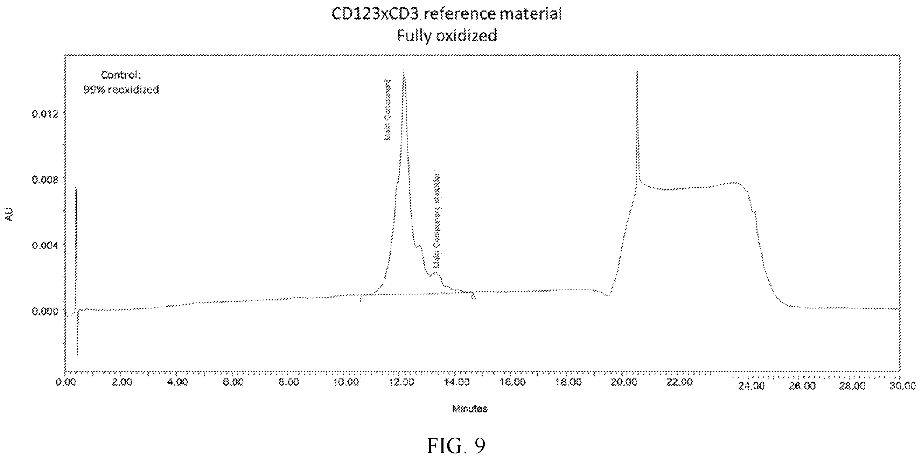

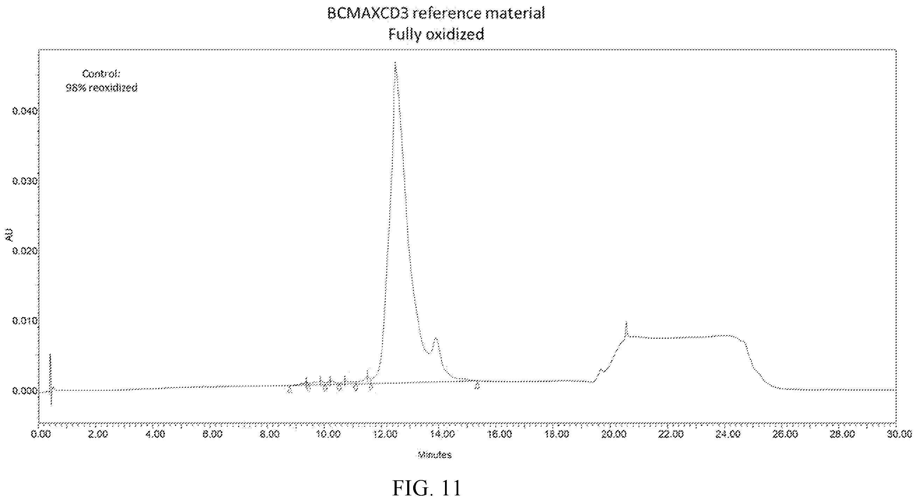

Integration of main component: the reference material main component was comprised of 2 peaks, which included a main component peak and main component shoulder peak (FIGS. 3, 5, 7, 9, and 11). These 2 peaks were integrated together between the valley of the last pre-peak and main peak and ending between the valley of the shoulder main peak and the first post-peak (FIGS. 3, 5, 7, 9, and 11). The pre-peak(s) and post-peak(s) to the main peak and main shoulder peak were comprised of unoxidized fragments and/or residual parental homodimer bispecific antibody that were contained in the reference material (control).

Integration of the pre-peak in the reference material (RM) was integrated as 1 peak. Integration of the post-peak in the reference material (RM) was integrated as 1 peak.

### Peak Integration of post cFAE test sample

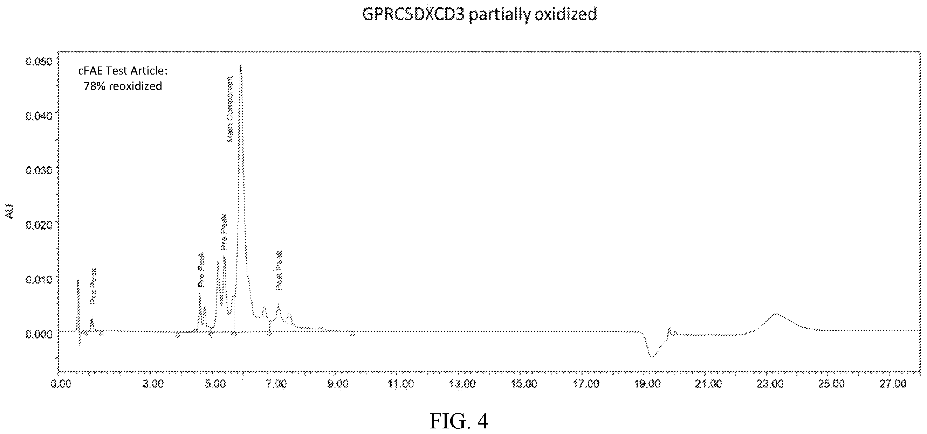

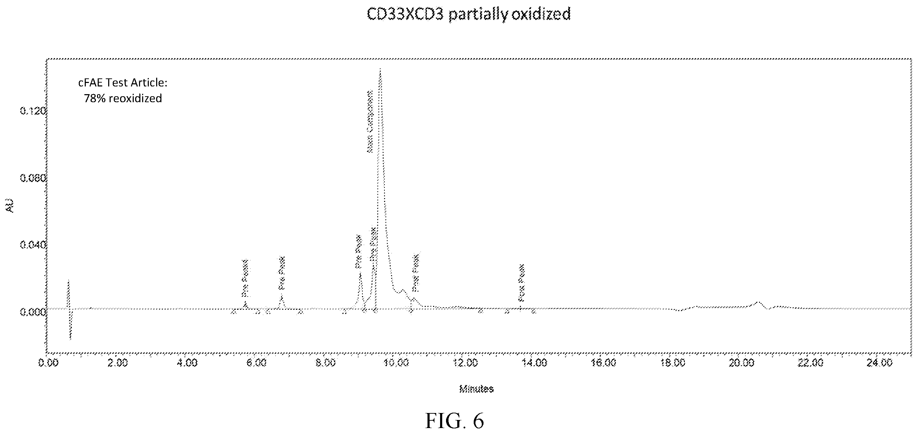

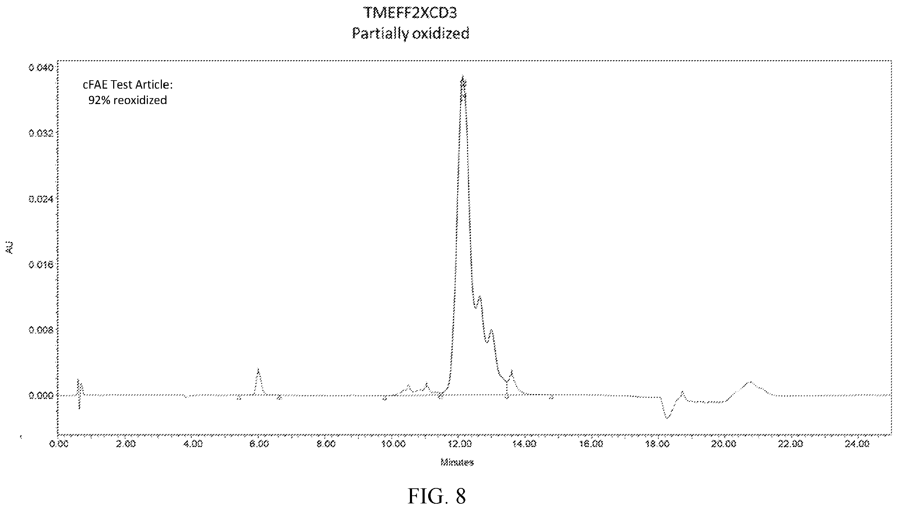

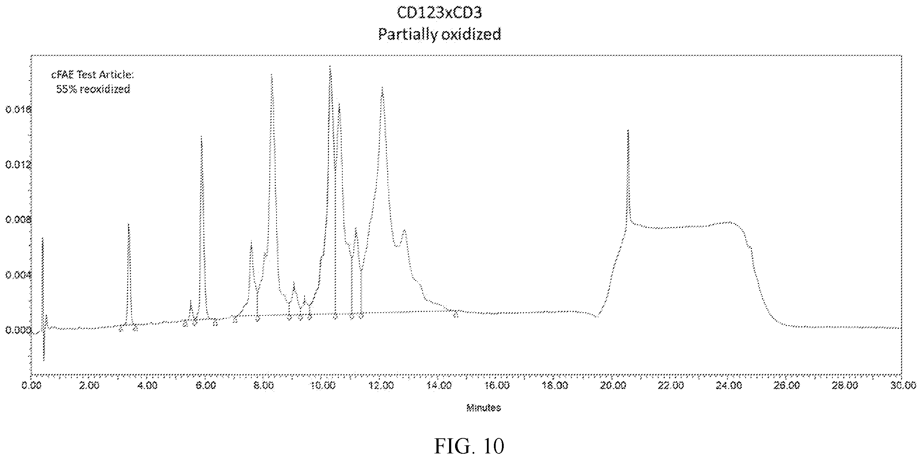

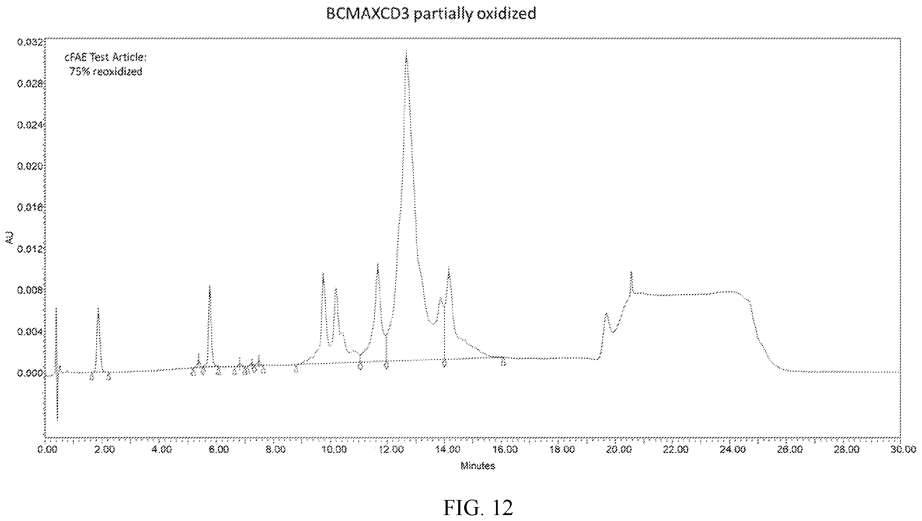

Integration of the post cFAE test article main component: the post cFAE test bispecific antibody was comprised of 2 peaks, which included the main component peak and main component shoulder peak (FIGS. 4, 6, 8, 10, and 12). These 2 peaks were integrated together between the valley of the last pre-peak and main peak and ending between the valley of the shoulder main peak and the first post-peak (FIGS. 4, 6, 8, 10, and 12).

Non baseline resolved pre-peak clusters prior to the main component were integrated together as a single pre-peak and individual baseline resolved peaks were integrated separately. All post-peaks after the main component shoulder peak were integrated as 1 peak. The pre- and post-peaks represent the unoxidized bispecific species (FIGS. 4, 6, 8, 10, and 12).

Peak identification for reference material: The 2 peaks which correspond to the bispecific antibody reference material were labeled as main component and main component shoulder peak. The multiple cluster peaks prior to the bispecific antibody were integrated together (FIGS. 3-12) and labeled as pre-peaks. The post-peaks to main component and main component shoulder peak were integrated together (FIGS. 3-12) and labeled as post-peaks.

It will be appreciated by those skilled in the art that changes could be made to the embodiments described above without departing from the scope of the claims.

## Claims

1. A method of detecting formation of a multi-specific molecule, the method comprising:
a. obtaining a multi-specific molecule sample;
b. obtaining a reversed-phase high performance liquid chromatography (RP-HPLC) column;
c. contacting the multi-specific molecule sample in a polar aqueous mobile phase A with the RP-HPLC column, wherein the polar aqueous mobile phase A is a solution comprising about 1% to about 5% isopropanol and an ion-pairing agent;
d. contacting an organic non-polar mobile phase B with the RP-HPLC column, wherein the organic non-polar mobile phase B is a solution comprising about 60% to about 80% isopropanol and about 15% to about 25% acetonitrile and an ion-pairing agent;
e. eluting the multi-specific molecule sample; and
f. monitoring the amount of multi-specific molecule formation in the eluted multispecific molecule sample.

2. The method of claim 1, wherein the polar aqueous mobile phase A is a solution comprising about 2% isopropanol.

3. The method of claim 1 or 2, wherein the ion-pairing agent in the polar aqueous mobile phase A is selected from the group consisting of trifluoroacetic acid (TFA), difluoroacetic acid (DFA), and formic acid (FA).

4. The method of claim 3, wherein the TFA is present in the polar aqueous mobile phase A at about 0.1% to about 2%.

5. The method of claim 4, wherein the TFA is present in the polar aqueous mobile phase A at about 0.1%.

6. The method of any preceding claim, wherein the organic non-polar mobile phase B is a solution comprising about 70% isopropanol.

7. The method of any preceding claim, wherein the organic non-polar mobile phase B is a solution comprising about 20% acetonitrile.

8. The method of claim 6 or 7, wherein the ion-pairing agent in the organic non-polar mobile phase B is selected from the group consisting of trifluoroacetic acid (TFA), difluoroacetic acid (DFA), and formic acid (FA).

9. The method of claim 8, wherein the TFA is present in the organic non-polar aqueous mobile phase B at about 0.1% to about 2%.

10. The method of claim 9, wherein the TFA is present in the organic non-polar aqueous mobile phase B at about 0.1%.

11. The method of any preceding claim, wherein the multi-specific molecule is a multispecific antibody and the method further comprises performing a manufacturing process to produce the multi-specific antibody, wherein the manufacturing process results in a multispecific antibody sample comprising an amount of the multi-specific antibody.

12. The method of claim 11, wherein the manufacturing process to produce a multispecific antibody is selected from the group consisting of a knob in hole process, a strand exchange engineered domain process, a chemical linked bispecific antibody (BsAb) process, an immunoglobulin domain crossover process, and a controlled Fab arm exchange (cFAE) and dual variable domain process.

13. The method of claim 11, wherein the multi-specific antibody is a bispecific antibody.

14. The method of any one of claims 1 to 10, wherein the multi-specific molecule is a bispecific antibody and the method further comprises performing a controlled FAB arm exchange (cFAE) to produce a cFAE sample, wherein the cFAE sample comprises an amount of the bispecific antibody.

15. A system comprising a reversed-phase high performance liquid chromatography (RP-HPLC) member for detecting formation of a multi-specific molecule, the system comprising:
a. a multi-specific molecule sample in a polar aqueous mobile phase A, wherein the polar aqueous mobile phase A is a solution comprising about 1% to about 5% isopropanol and an ion pairing agent;
b. a RP-HPLC column;
c. an organic non-polar mobile phase B, wherein the organic non-polar mobile phase B is a solution comprising about 60% to about 80% isopropanol and about 15% to about 25% acetonitrile and an ion pairing agent;
wherein the RP-HPLC column is configured to be contacted with the multi-specific molecule sample in polar aqueous mobile phase A, and
wherein the RP-HPLC is further configured to be contacted with the organic non-polar mobile phase B to elute a multi-specific molecule sample, wherein an amount of multispecific molecule formation can be identified in the eluted multi-specific molecule sample.

## Patentansprüche

1. Verfahren zum Nachweisen der Bildung eines multispezifischen Moleküls, das Verfahren umfassend:
a. Erhalten einer multispezifischen Molekülprobe;
b. Erhalten einer Hochleistungsflüssigkeitschromatographie-Säule (RP-HPLC) mit veränderter Phase;
c. Inkontaktbringen der multispezifischen Molekülprobe in einer polaren wässrigen mobilen Phase A mit der RP-HPLC-Säule, wobei die polare wässrige mobile Phase A eine Lösung ist, die etwa 1 % bis etwa 5 % Isopropanol und ein lonenpaarungsmittel umfasst;
d. Inkontaktbringen einer organischen unpolaren mobilen Phase B mit der RP-HPLC-Säule, wobei die organische unpolare mobile Phase B eine Lösung ist, die etwa 60 % bis etwa 80 % Isopropanol und etwa 15 % bis etwa 25 % Acetonitril und ein lonenpaarungsmittel umfasst;
e. Eluieren der multispezifischen Molekülprobe; und
f. Überwachen der Menge der multispezifischen Molekülbildung in der eluierten multispezifischen Molekülprobe.

2. Verfahren nach Anspruch 1, wobei die polare wässrige mobile Phase A eine Lösung ist, die etwa 2 % Isopropanol umfasst.

3. Verfahren nach Anspruch 1 oder 2, wobei das Ionenpaarungsmittel in der polaren wässrigen mobilen Phase A aus der Gruppe, bestehend aus Trifluoressigsäure (TFA), Difluoressigsäure (DFA) und Ameisensäure (FA), ausgewählt wird.

4. Verfahren nach Anspruch 3, wobei das TFA in der polaren wässrigen mobilen Phase A zu etwa 0,1 % bis etwa 2 % vorhanden ist.

5. Verfahren nach Anspruch 4, wobei das TFA in der polaren wässrigen mobilen Phase A zu etwa 0,1 % vorhanden ist.

6. Verfahren nach einem der vorstehenden Ansprüche, wobei die organische unpolare mobile Phase B eine Lösung ist, die etwa 70 % Isopropanol umfasst.

7. Verfahren nach einem der vorstehenden Ansprüche, wobei die organische unpolare mobile Phase B eine Lösung ist, die etwa 20 % Acetonitril umfasst.

8. Verfahren nach Anspruch 6 oder 7, wobei das Ionenpaarungsmittel in der organischen unpolaren mobilen Phase B aus der Gruppe, bestehend aus Trifluoressigsäure (TFA), Difluoressigsäure (DFA) und Ameisensäure (FA), ausgewählt ist.

9. Verfahren nach Anspruch 8, wobei das TFA in der organischen, unpolaren, wässrigen mobilen Phase B zu etwa 0,1 % bis etwa 2 % vorhanden ist.

10. Verfahren nach Anspruch 9, wobei das TFA in der organischen, unpolaren, wässrigen mobilen Phase B zu etwa 0,1 % vorhanden ist.

11. Verfahren nach einem der vorstehenden Ansprüche, wobei das multispezifische Molekül ein multispezifischer Antikörper ist und das Verfahren außerdem die Durchführung eines Herstellungsprozesses zur Herstellung des multispezifischen Antikörpers umfasst, wobei der Herstellungsprozess zu einer multispezifischen Antikörperprobe führt, die eine Menge des multispezifischen Antikörpers umfasst.

12. Verfahren nach Anspruch 11, wobei das Herstellungsverfahren zur Produktion eines multispezifischen Antikörpers aus der Gruppe ausgewählt wird, bestehend aus einem Knopf-in-Loch-Verfahren, einem Strangaustauschverfahren, einem chemisch gebundenen bispezifischen Antikörper (BsAb), einem Immunglobulin-Domänen-Crossover-Verfahren und einem kontrollierten Fab-Arm-Austausch (cFAE) und einem dualen variablen Domänenprozess.

13. Verfahren nach Anspruch 11, wobei der multispezifische Antikörper ein bispezifischer Antikörper ist.

14. Verfahren nach einem der Ansprüche 1 bis 10, wobei das multispezifische Molekül ein bispezifischer Antikörper ist und das Verfahren ferner die Durchführung eines kontrollierten FAB-Armaustauschs (cFAE) umfasst, um eine cFAE-Probe herzustellen, wobei die cFAE-Probe eine Menge des bispezifischen Antikörpers umfasst.

15. System, das ein Element der Umkehrphasen-Hochleistungsflüssigkeitschromatographie (RP-HPLC) zum Nachweisen der Bildung eines multispezifischen Moleküls umfasst, das System umfassend:
a. eine multispezifische Molekülprobe in einer polaren wässrigen mobilen Phase A, wobei die polare wässrige mobile Phase A eine Lösung ist, die etwa 1 % bis etwa 5 % Isopropanol und ein Ionenpaarungsmittel umfasst;
b. eine RP-HPLC-Säule;
c. eine organische unpolare mobile Phase B, wobei die organische unpolare mobile Phase B eine Lösung ist, die etwa 60 % bis etwa 80 % Isopropanol und etwa 15 % bis etwa 25 % Acetonitril und ein Ionenpaarungsmittel umfasst;
wobei die RP-HPLC-Säule konfiguriert ist, um mit der multispezifischen Molekülprobe in polarer wässriger mobiler Phase A in Kontakt gebracht zu werden, und
wobei die RP-HPLC ferner konfiguriert ist, um mit der organischen unpolaren mobilen Phase B in Kontakt gebracht zu werden, um eine multispezifische Molekülprobe zu eluieren, wobei eine Menge der multispezifischen Molekülbildung in der eluierten multispezifischen Molekülprobe identifiziert werden kann.

## Revendications

1. Procédé de détection de formation d'une molécule multispécifique, le procédé comprenant :
a. l'obtention d'un échantillon de molécule multispécifique ;
b. l'obtention d'une colonne de chromatographie liquide haute performance en phase inversée (RP-HPLC) ;
c. la mise en contact de l'échantillon de molécule multispécifique dans une phase mobile aqueuse polaire A avec la colonne RP-HPLC, dans lequel la phase mobile aqueuse polaire A est une solution comprenant environ 1 % à environ 5 % d'isopropanol et un agent d'appariement d'ions ;
d. la mise en contact d'une phase mobile non polaire organique B avec la colonne RP-HPLC, dans lequel la phase mobile non polaire organique B est une solution comprenant environ 60 % à environ 80 % d'isopropanol et environ 15 % à environ 25 % d'acétonitrile et un agent d'appariement d'ions ;
e. l'élution de l'échantillon de molécule multispécifique ; et
f. la surveillance de la quantité de formation de molécule multispécifique dans l'échantillon de molécule multispécifique élué.

2. Procédé selon la revendication 1, dans lequel la phase mobile aqueuse polaire A est une solution comprenant environ 2 % d'isopropanol.

3. Procédé selon la revendication 1 ou 2, dans lequel l'agent d'appariement d'ions dans la phase mobile aqueuse polaire A est choisi dans le groupe constitué d'acide trifluoroacétique (TFA), acide difluoroacétique (DFA), et acide formique (FA).

4. Procédé selon la revendication 3, dans lequel le TFA est présent dans la phase mobile aqueuse polaire A à raison d'environ 0,1 % à environ 2 %.

5. Procédé selon la revendication 4, dans lequel le TFA est présent dans la phase mobile aqueuse polaire A à raison d'environ 0,1 %.

6. Procédé selon l'une quelconque revendication précédente, dans lequel la phase mobile non polaire organique B est une solution comprenant environ 70 % d'isopropanol.

7. Procédé selon l'une quelconque revendication précédente, dans lequel la phase mobile non polaire organique B est une solution comprenant environ 20 % d'acétonitrile.

8. Procédé selon la revendication 6 ou 7, dans lequel l'agent d'appariement d'ions dans la phase mobile non polaire organique B est choisi dans le groupe constitué d'acide trifluoroacétique (TFA), acide difluoroacétique (DFA), et acide formique (FA).

9. Procédé selon la revendication 8, dans lequel le TFA est présent dans la phase mobile aqueuse non polaire organique B à raison d'environ 0,1 % à environ 2 %.

10. Procédé selon la revendication 9, dans lequel le TFA est présent dans la phase mobile aqueuse non polaire organique B à raison d'environ 0,1 %.

11. Procédé selon l'une quelconque revendication précédente, dans lequel la molécule multispécifique est un anticorps multispécifique et le procédé comprend en outre la réalisation d'un processus de fabrication pour produire l'anticorps multispécifique, dans lequel le processus de fabrication aboutit à un échantillon d'anticorps multispécifique comprenant une quantité de l'anticorps multispécifique.

12. Procédé selon la revendication 11, dans lequel le processus de fabrication pour produire un anticorps multispécifique est choisi dans le groupe constitué d'un processus « nœud dans la cavité », un processus de domaine modifié par échange de brins, un processus d'anticorps bispécifique (BsAb) lié chimiquement, un processus de croisement de domaines d'immunoglobuline, et un processus d'échange contrôlé de bras Fab (cFAE) et de double domaine variable.

13. Procédé selon la revendication 11, dans lequel l'anticorps multispécifique est un anticorps bispécifique.

14. Procédé selon l'une quelconque des revendications 1 à 10, dans lequel la molécule multispécifique est un anticorps bispécifique et le procédé comprend en outre la réalisation d'un échange contrôlé de bras FAB (cFAE) pour produire un échantillon de cFAE, dans lequel l'échantillon de cFAE comprend une quantité de l'anticorps bispécifique.

15. Système comprenant un élément de chromatographie liquide haute performance en phase inversée (RP-HPLC) permettant de détecter la formation d'une molécule multispécifique, le système comprenant :
a. un échantillon de molécule multispécifique dans une phase mobile aqueuse polaire A, dans lequel la phase mobile aqueuse polaire A est une solution comprenant environ 1 % à environ 5 % d'isopropanol et un agent d'appariement d'ions ;
b. une colonne RP-HPLC ;
c. une phase mobile non polaire organique B, dans lequel la phase mobile non polaire organique B est une solution comprenant environ 60 % à environ 80 % d'isopropanol et environ 15 % à environ 25 % d'acétonitrile et un agent d'appariement d'ions ;
dans lequel la colonne RP-HPLC est conçue pour être mise en contact avec l'échantillon de molécule multispécifique dans la phase mobile aqueuse polaire A, et
dans lequel la RP-HPLC est en outre conçue pour être mise en contact avec la phase mobile non polaire organique B pour éluer un échantillon de molécule multispécifique, dans lequel une quantité de formation de molécule multispécifique peut être identifiée dans l'échantillon de molécule multispécifique élué.
